# EUROPEAN PATENT SPECIFICATION

(11) **EP 2 493 448 B1**
(45) Date of publication and mention of the grant of the patent: **08.10.2014**
(21) Application number: 10778540.4
(22) Date of filing: 27.10.2010
(51) Int. Cl.: A61K 8/58, A61Q 1/08

(54) **COSMETIC COMPOSITION COMPRISING A SILICONED SAPUCAINHA ESTER**
KOSMETISCHE ZUSAMMENSETZUNG ENTHALTEND EINEN SILIKONIERTEN SAPUCAINHA ESTER
COMPOSITION COSMETIQUE COMPRENANT UN ESTER SILICONE DE SAPUCAINHA

(30) Priority: 27.10.2009 US 606278
(43) Date of publication of application: 05.09.2012
(73) Proprietor: Natura Cosméticos S.A., CEP-06882-700 Itapecerica da Serra, SP (BR)
(72) Inventor: DADARIO BRUGNOLLO, Erica, 05590-130 - Vila Gomes - SP (BR); SCARPARO DE SANCTIS, Daisy de Fatima, 04704-140 - Brooklin - SP (BR); MORAES SANTOS, Leandra, 13073-090 - Sao Paulo - SP (BR); LORENCINI, Marcio, 13084-551 - Campinas - SP (BR); MOURA SA ROCHA, Vanessa de, 01420-002 - Sao Paulo - SP (BR); FIGUEIREDO BEDA, Debora, 05376-140 - Sao Paulo - SP (BR)
(74) Representative: Brady, Paul Andrew
(86) International application number: PCT/BR2010/000354
(87) International publication number: WO 2011/050433

(56) References cited:
- WO-A2-2006/045932
- FR-A1- 2 706 304
- FR-A1- 2 876 908
- FR-A1- 2 876 909
- US-A- 3 563 941
- DATABASE GNPD [Online] Mintel; 1 December 2003 (2003-12-01), "Exfoliating bath soap", XP002621859, Database accession no. 10156342
- OLIVEIRA A S ET AL: "Acidos ciclopentenicos do oleo da sapucainha (Carpotroche brasiliensis Endl, Flacourtiaceae): O primeiro antileprotico usado no brasil [Cyclopentenyl acids from sapucainha oil (Carpotroche brasiliensis Endl, Flacourtiaceae): the first antileprotic used in Brazil]", QUIMICA NOVA, vol. 32, no. 1, 1 February 2009 (2009-02-01), pages 139-145, XP008109750, SOCIEDADE BRASILEIRA DE QUIMICA, SAO PAULO, BR ISSN: 0100-4042, DOI: :10.1590/S0100-40422009000100027 [retrieved on 2009-01-20]
- NORTON ET AL: "Useful plants of dermatology. I. Hydnocarpus and chaulmoogra", JOURNAL OF THE AMERICAN ACADEMY OF DERMATOLOGY, vol. 31, no. 4, 1 October 1994 (1994-10-01), pages 683-686, XP025763704, C.V. MOSBY, ST. LOUIS, MO, US ISSN: 0190-9622, DOI: 10.1016/S0190-9622(08)81744-6 [retrieved on 1994-10-01]

## Description

### FIELD OF THE INVENTION

The present invention relates to cosmetic composition comprising siliconed sapucainha ester, compound which can be used as a cosmetic excipient replacing silicones for several applications, as well as to products comprising said composition.

### BACKGROUND OF THE INVENTION

In cosmetic field, there are known various applications for silicones and/or silicone derivatives in combination with lipidic components of plant origin. Among the typical examples of these applications, it can be mentioned photoprotection, spreadability agents, surfactants, consistency and rheological agents, among others. However, the compositions disclosed in the literature do not specifically describe silicone ester compounds.

Siloxanes are silicone derivatives having excellent properties as spreadability agents in most of the substrates. These materials are widely used in cosmetic industry as mentioned above.

Skin aging has been defined as a process of accumulated molecular damage through time (Giacomoni, P.U., Aging and Cellular Defense Mechanisms. Ann. NY Acad. Sci. 1992; 663:1-3). One of the most visible signs of aging is the change in the aspect of the skin, which begins to show wrinkles, flaccidity, spots, loss of elasticity and thickness (Yaar, M. & Gilchrest, B.A., Aging of Skin. In: Freedberg IM, Eisen AZ, Wolf K, Austen KF, Goldsmith LA, Katz SI (eds.). Fitzpatrick's Dermatology in General Medicine, volume 1, 6th ed., McGraw-Hill, 2003; 1386-1398). Aging is a complex process the mechanisms of which can be caused by internal and/or external factors.

The external factors are related with ultraviolet radiation (UV), diet and other factors related to the daily routine, such as life habits. The internal factors, on the other hand, are either genetically controlled or derive from processes resulting from genetically unprogrammed transformations called epigenetic transformations (Yaar & Gilchrest, 2003).

Recently, scientific studies have investigated the action of these factors in the skin aging process and have shown that, in addition to direct damages caused to the skin macromolecules, another important process acts on aging. The constant exposure to sunlight, for instance, induces a process called skin micro-inflammation, which acts constantly on the skin (Giacomoni, P.U., Declercq, L., Hellemans, L., Maes, D. Aging of Human Skin: review of a mechanistic model and first experimental data. IUBMB Life 2000; 49: 259-263).

Unlike classic inflammation, in which quite characteristic clinical signs are observed, such as redness, heat and edema, micro-inflammation does not produce these signs. Skin micro-inflammation is imperceptible and produces signs that characterize skin aging (specially in the face and other skin areas under constant UV exposure), because, latently, micro-inflammation induces the migration of pro-inflammatory cells is small number, which then induce the expression of cytokines and enzymes that are involved in the constant degradation of the extracellular matrix (ECM) culminating in loss of firmness, loss of skin elasticity and the appearance and deepening of wrinkles (Giacomoni, P.U., Rein, G., A Mechanistic Model For the Aging of Human Skin. Micron 2004; 35: 179-184; and Chung, H.Y., Kim, H.J., Kim, K.W., Choi, J.S., Yu, B.P., Molecular Inflammation Hypothesis of Aging Based on the Anti-Aging Mechanism of Calorie Restriction. Microsc Res Tech. 2002; 59: 264-272). Among the enzymes secreted, a family called matrix metalloproteinases (MMPs) us noteworthy, with the ability to degrade ECM structural proteins, such as collagen, elastin, laminin, fibronectin and proteoglycans (Sternlicht & Werb, 2003).

The theory based on the skin micro-inflammatory processes has gained considerable importance in the cosmetic area, being a new target mechanism for anti-aging products (Draelos, Z.D., Innovations in Dermatology: Rethinking the Aging Face. Cosmetics & Toiletries Magazine 2005; 120: 75-78; Giacomoni, P.U., Aging, Science and the cosmetics industry. EMBO Reports 2005; 6: S45-S48; Iddamalgoda, A., Ito, K., Tanaka, K., Banno, N., Tsuboi, T., Kojima, H., Possible Involvement of Mast Cell Tryptase in Extra Cellular Matrix Damage: Implications on Anti-Aging Skin Care. In: Abstract Book of the 24th IFSCC Congress, Osaka, Japan, 2006: 118-119; and Seo, M.Y., Chung, S.Y., Choi, W.K., Ryu, B.H., Park, J.M., Seo, M.J., Kim, J.W., Rice Wine May Protect Skin From UV-Induced Inflammation and Delay Skin Aging. In: Abstract Book of the 24th IFSCC Congress, Osaka, Japan, 2006: 120-121). This model results from the observation that extrinsic aging processes, such as UV radiation, stimulate the synthesis of adhesion molecules that facilitate the entrance of inflammatory cells (including monocytes and granulocytes) into the skin (Giacomoni, 2000). Under these conditions, neighboring resident cells, such as fibroblasts and keratinocytes, are likely to be affected by the free radicals, also increasing their release of cytokines and enzymes.

Actually, any aggressive external stimulus acts on the release of inflammatory mediators by macrophages, lymphocytes, fibroblasts and keratinocytes (Dasu, M.R.K., Barrow, R.E., Spies, M., Herndon, D.N., Matrix Metalloproteinase Expression in Cytokine Stimulated Human Dermal Fibroblasts. Burns 2003; 29: 527-531). After stimulus, there is the induction of a series of cytokines including TNF-alpha, Interleukin-1 (IL-1), Interleukin-6 (IL-6) and Interleukin-8 (IL-8), which are capable of stimulating the production of MMPs by skin fibroblasts (Dasu et al., 2003; Fang, Q., Liu, X., Al-Mugotir, M., Kobayashi, T., Abe, S., Kohyama, T., Rennard, S.I., Thrombin and TNF-alfa/IL-1 beta Synergistically Induce Fibroblast Mediated Collagen Gel Degradation. AJRCMB 2006; 1-46; and Wisithphrow, K., Murray, P.E., Windsor, L.J., Interleukin-1 Alpha Alters the Expression of Matrix Metalloproteinases and Collagen Degradation by Pulp Fibroblasts. J Endod 2006; 32: 186-192). The enzymes released may act on EMC degradation, accelerating the skin aging process. In addition, some cytokines can cause a small reduction in the release of MMP inhibitors (Fang et al., 2006) further contributing to matrix degradation. Since MMP secretion reaches high levels and the secretion of its inhibitors is reduced or does not increase in the same proportion, molecular imbalance results and this is evidenced in the skin through the formation of expression wrinkles that accumulate with time in individuals (Oh, J.H., Kim, A., Park, J.M., Kim, S.H., Chung, A.S., Ultraviolet B-induced Matrix Metallo-proteinase-1 and -3 Secretions Are Mediated Via PTEN/AKT Pathway in Human Dermal Fibroblasts. J Cell Physiol., 2006; 209: 775-785).

The documents of the state of the art below are related to compositions comprising siloxanes in combination with vegetal agents.

US 20040228811**:** shows a solar filter formulation comprising about 53.5% to about 98.7% water, about 1% to about 40% of solar filter active, about 0.1% to about 0.5% of Pemulen TR-2, about 0.1 to about 5% of emulsifying stabilizer, and about 0.1 % to about 1% of neutralizing agent being all concentrations based on the total weight of the formulation. In this application, a trisiloxane is cited as an absorber of ultraviolet rays (UV) and vegetal components are cited as emulsifiers.

US 20050207999**:** discloses a sunscreen composition comprising, as a sunscreen component, a trisiloxane, and a grease phase, a wax that may have animal origin.

Many other documents in which a composition comprising a component derived from silicone or a silicone in combination with lipidic components, such as US 2005239669, US 200600572117, US 2006140924, US 20050169876, GB 1140 536, US 5482703 and FR 2771003, can be cited here for reference.

### SUMMARY OF THE INVENTION

The present invention relates to a cosmetic composition comprising siliconed sapucainha ester, compound which can be used as a cosmetic excipient replacing silicones for several applications.

In addition, the present invention relates to cosmetic products comprising said composition.

### BRIEF DESCRIPTION OF DRAWING

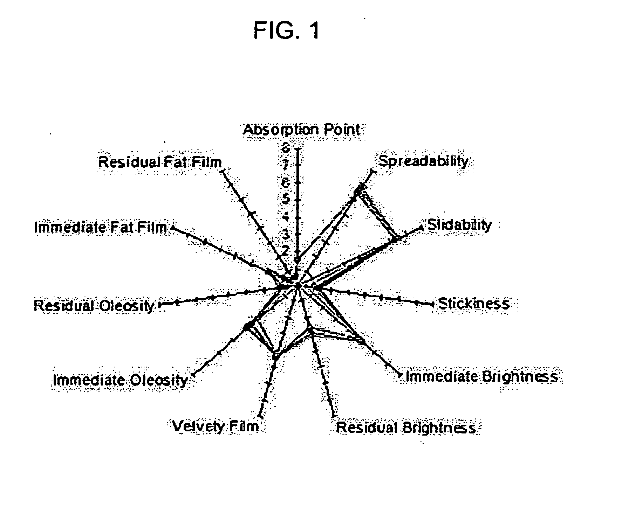

Figure 1: Graphic representation of the results obtained in test 2 referring to the application of the siliconed sapucainha ester in known formulations such as that of product *Chronos Flavonóides de Passiflora 30 + Dia.*

### DETAILED DESCRIPTION OF THE INVENTION

The chaulmoogra oil has been known and used for centuries with therapeutic purposes and several documents in the literature describe its specific application for treating leprosy, and its use in cosmetic compositions.

The chaulmoogra oil may be extracted, among other species, from plants of species popularly known in Brazil by the names sapucainha, papo de anjo, pau de cachimbo, pau de lepra, and others. The oil is normally extracted from the sapucainha seeds and its topical cosmetic use has been studied.

These plants are scientifically known as *Carpotroche brasiliensis.*

It has now been surprisingly found that compositions comprising ester obtained from sapucainha comprising silicone, that is, the heptamethyltriloxane-3-ethylcyclohexanol-(1-cyclopentyl)-hexadecanoate ester, having the following formula, has interesting characteristics for use in compositions for topic application as cosmetic application.

In accordance with a preferred embodiment of the invention, a siliconed sapucainha ester described in patent document "Silicone Modified Fatty Acids, Method of Preparation and Usage Thereof" (US 20100040570), is used.

Preferably, said ester is the heptamethyltrisiloxane-3-ethylcyclohexanol-(1-cyclopentenyl)-hexadecanoate ester of the formula:

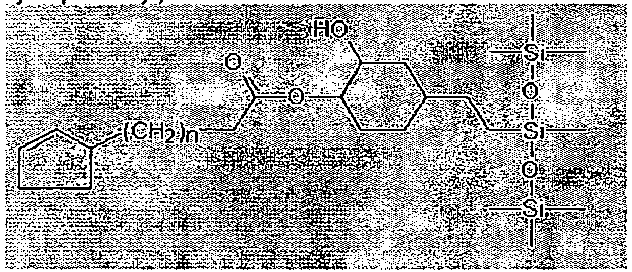

Firstly, fatty acids are obtained from sapucainha butter preferably from *Carpotroche brasiliensis.*

The sapucainha butter basically consists of triglycerides that are extracted from almonds containing 90% of fat with high concentration of long chain fatty acids (hydnocarpic, chaulmoogric and gorlic) and melting point at 32°C. For obtaining the butter, the seeds are pressed. The mechanical pressing mechanism is a physical process and more environmentally correct because it prevents the use of organic solvents for the chemical extraction of oil. The sapucainha butter is also submitted to the refining process, clarification and deodorization and then enzymatic hydrolysis for the production of sapucainha fatty acid. The conjugation with silicone is carried out with the fatty acid so as to maintain the vegetal property of the molecule.

The obtainment of fatty acids from sapucainha butter can be made through the chemical or enzymatic route. The butter is preferably refined before the beginning of the process, that is, it is clarified and deodorized.

Preferably, the afore-mentioned ester is prepared according to the method described in patent document "Silicone Modified Fatty Acids, Method of Preparation and Usage Thereof" (official number not yet available).

The cosmetic compositions of this invention may include, preferably, from 0,1% to 5,0% by weight of siliconed sapucainha ester and most preferably 0,1% to 3,0% by weight, based on the total weight of the composition, and a cosmetically acceptable vehicle.

The composition of the present invention can be illustrated by the following examples 1 to 4:

| Raw material | Example 1 | Example 2 | Example 3 | Example 4 |
|---|---|---|---|---|
| 1. Demineralized water | 77.750 | 76.650 | 74.750 | 77.700 |
| 2. Disodium EDTA | 0.100 | 0.100 | 0.100 | 0.100 |
| 3. Bidistilled Glycerin Bxr Vegetable | 5.000 | 5.000 | 5.000 | 5.000 |
| 4.Hydroxyethyl cellulose | 0.100 | 0.200 | 0.100 | 0.150 |
| 5. Cetostearyl alcohol | 4.000 | 3.000 | 2.000 | 3.500 |
| 6. Cetyl alcohol | 1.000 | 1.000 | 2.000 | 0.500 |
| 7.Siliconed sapucainha ester | 1.000 | 2.500 | 4.000 | 0.500 |
| 8. Cetyltrimethylammonium chloride | 1.000 | 1.500 | 2.000 | 2.500 |
| 9.Demineralized water | 10.000 | 10.000 | 10.000 | 10.000 |
| 10. Preservative | 0.050 | 0.050 | 0.050 | 0.050 |

The compositions of the present invention are not limited only to the vehicles and excipients described above, but they can also include other physiologic acceptable vehicles and excipients.

The use of the cosmetic composition of this invention is indicated particularly to hygienic or aesthetic benefit to the human body.

Non-limiting examples of cosmetic products that can be prepared comprising siliconed sapucainha ester as described above are:
❖ Scalp products;
❖ Sunscreen or Sun blockers for adult or child use, for concomitant practice of sports or not;
❖ Post-sun products;
❖ Body or facial moisturizers;
❖ Body or facial anti-aging products;
❖ Body or facial firming lotions;
❖ Self-tanning products;
❖ Skin brightening body or facial moisturizers;
❖ Creams for hands and feet for intensive moisturizing of regions with cracks;
❖ Deodorants;
❖ Tonics;
❖ Depilatory creams;
❖ Cleansing lotion;
❖ Products for hair with high-conditioning: conditioners, hydration mask, leave-on, leave-in;
❖ Foot talc;
❖ Anti-cellulite products;
❖ Aftershave products;
❖ Body or facial cosmetic preparations for child use;
❖ Cosmetic preparations for localized action, specific to periocular region, contour of the lips, lips, blemishes, under-eye circles, among others;
❖ Antiacne products;
❖ Skin clarifying products;
❖ Lipsticks and waxy bases;
❖ Blushers and tinted bases;
❖ Facial powders;
❖ Any makeup product for the eye area.

The formulation possibilities are countless since the siliconed sapucainha ester exhibits hydrating action, is capable of dispersing dyes, promotes barrier protection/film-formation, being able to substitute synthetic silicones, such as cyclomethicone, as well as to lead potentially to cost reduction of the final formula, In addition, said ester has a pale / amber yellow color, which does not influence the color of the final product and, as it gives adequate sensory (texture, softness, film, brightness, appropriate slideness), it is easy to be added to the formulas and it increases the content of plant source compounds in the compositions.

### Test 1: Evaluation of the surface tension of the siliconed sapucainha ester through two different methods.

### Method 1: Maximum bubble pressure method (Jaeger method)

| Product | Surface Tension (dynes/cm) | T (°C) |
|---|---|---|
| Siliconed sapucainha ester | 38.5 | 56.6 |
| Sapucainha fatty acid | 43.1 | 56.5 |
| Sapucainha butter | 47.8 | 55.8 |

### Method 2: Wilhelmy plate method

| **Samples** | **STF(mN/M)** |
|---|---|
| Ethanol | 21.959 |
| Siliconed sapucainha ester | 22.134 |
| Mixture of cyclic silicones mainly composed of cyclopentasiloxane (100% silicone fluid) | 20.857 |
| Palm Olein PN3 | 32.616 |

The modified sapucainha fatty ester has lower surface tension (lower than that of the pure fatty acid and that of the sapucainha butter). It is known that the lower the surface tension, the higher the spreadability. Therefore, based on these tests, it is verified that the spreadability of the siliconed sapucainha ester is higher than that of the fatty acids and butter, being a desired attribute in cosmetic compositions.

As will be seen in test 4 described below, a better spreadability of the siliconed sapucainha ester is highly important because it imparts sensory similar to that of silicone.

### Test 2: Application of the siliconed sapucainha ester in known formulations such as that of product Chronos Flavonóides de Passiflora 30+ Dia.

In this study, 3% of the crosspolymer commonly used in the formula of product *Chronos Flavonóides de Passiflora 30+ Dia* was replaced with 1 to 5% siliconed sapucainha ester. During the study, it was noted that the formulation containing 1% of said ester had a rheological behavior similar to that observed with 3% of the crosspolymer commonly used and that the formulation containing 5% of said ester had an increase in thickness. Therefore, the tests continued by replacing said crosspolymer with only 1 to 3% of the ester.

A comparison was made through a descriptive sensory analysis of the four formulations using the Tukey's Test (for multiple comparison), in order to better evaluate the effects of the substitution in question:
P01 - *Chronos Flavonóides de Passiflora 30+ Dia*
P02 - Formula containing 3% of the siliconed sapucainha ester;
P03 - Formula containing 1% of the siliconed sapucainha ester;
P04 - Placebo.

The analysis was carried out by Institute Perception with 2 trained panelists, who were selected by said institute according to predetermined criteria of discrimination, repeatability and agreement.

The products were evaluated in circular sites of 5 cm in diameter in the forearm region, two fingers away from the wrists and two fingers away from the elbows. The amount of product applied in each site was 25 µL, spread in rotations, obeying the rhythm of the metronome at the speed of 120 pulses per minute.

The following attributes were assessed:
- Absorption Point: Number of rotations needed for the product to start being absorbed by the skin;
- Spreadability: Easiness to spread the product on the skin;
- Slidability: Easiness to spread / slide the finger on the skin;
- Immediate skin brightness: Light intensity reflected on the skin immediately after the product is spread;
- Residual skin brightness: Light intensity reflected on the skin two minutes after the product is spread;
- Stickiness: Intensity with which the finger adheres to the skin;
- Immediate oleosity: Oil sensation on the skin during and after the product is spread;
- Residual oleosity: Oil sensation on the skin two minutes after the product is spread;
- Immediate fat film: Fat sensation forming a film on the skin immediately after the product is spread;
- Residual fat film: Fat sensation forming a film on the skin two minutes after the product is spread;
- Velvety film: Peach skin sensation, and
- White residue: Formation of a white film on the skin.

And the following methodologies were adopted for evaluating the attributes:
- Absorption Point:
   The product is applied in one of the circular sites and the number of rotations needed for the product to start being absorbed by the skin is observed.
- Other Attributes:
   The product is applied in one of the circular sites and 15 rotations are done to spread it and make the evaluations (immediately or after one or two minutes).
   The following statistical analyses were carried out in the present study for each attribute, per product evaluated;
- Averages for each attribute per product assessed;
- ANOVA (causes of variation: panelist, sample and panelist x sample) followed by TUKEY's test);
- Polar Coordinates Graph with the averages of the attributes and
- Main Component Analysis

All the statistical analyses were carried out using software XLSTAT 2008, FIZZ 2.10f and MINITAB.

| Table - Averages and Results of the Tukey's Test - 5% | | | | |
|---|---|---|---|---|
| Attributes | P01 | P02 | P03 | P04 |
| Absorption Point | 1.48A | 1.47A | 1.45A | 1.51A |
| Spreadability | 6.39B | 6.54AB | 6.41AB | 6.64AB |
| Slidability | 6.31A | 6.44A | 6.34A | 6.50A |
| Stickiness | 1.01A | 1.05A | 1.16A | 1.32A |
| Immediate Brightness | 4.67A | 4.84a | 5.07A | 5.05A |
| Residual Brightness | 2.59A | 3.06a | 2.90Aa | 2.90A |
| Velvety Film | 4.46A | 4.38A | 4.24A | 4.36A |
| White Residue | 0.00A | 0.00A | 0.00A | 0.00A |
| Immediate Oleosity | 3.52AB | 3.76A | 3.85A | 3.46AB |
| Residual Oleosity | 0.88A | 0.90A | 1.06A | 0.83A |
| Immediate Fat Film | 1.73A | 1.54A | 1.70A | 1.75A |
| Residual Fat Film | 0.39A | 0.32AB | 0.39A | 0.35AB |

Averages followed by the same letter in each line do not significantly differ from each other in the specified significance level (Tukey's Test).

| Table - Averages and Results of the Tukey's Test - 10% | | | | |
|---|---|---|---|---|
| Attributes | P01 | P02 | P03 | P04 |
| Absorption Point | 1.48A | 1.47A | 1.45A | 1.51A |
| Spreadability | 6.39B | 6.54AB | 6.41 B | 6.64AB |
| Slidability | 6.31A | 6.44A | 6.34A | 6.50A |
| Stickiness | 1.01A | 1.05A | 1.16A | 1.32A |
| Immediate Brightness | 4.67A | 4.84a | 5.07A | 5.05A |
| Residual Brightness | 2.59A | 3.06a | 2.90Aa | 2.90A |
| Velvety Film | 4.46A | 4.38A | 4.24A | 4.36A |
| White Residue | 0.00A | 0.00A | 0.00A | 0.00A |
| Immediate Oleosity | 3.52AB | 3.76A | 3.85A | 3.46AB |
| ResidualOleosity | 0.88A | 0.90A | 1.06A | 0.83A |
| Immediate Fat Film | 1.73A | 1.54A | 1.70A | 1.75A |
| Residual Fat Film | 0.39A | 0.32AB | 0.39A | 0.35AB |

Averages followed by the same letter in each line do not significantly differ from each other in the specified significance level (Tukey's Test).

The representation of the results in graphic form can be seen in Figure 2.

Through the results presented in Figure 2, it is possible to see that there is no significant difference in the attributes assessed in the formulas (the products had no significant difference (p>0.05)). The attributes considered favorable (high spreadability and slidability, absorption point and low oily residue) for the cosmetic products of the Chronos line were evidenced in all formulas analyzed.

Thus, through the results obtained from the trained panel, it is concluded that 3% of the crosspolymer (synthetic silicone in the formulations of the *Chronos* line) can be substituted with 1% of the siliconed sapucainha ester (80% plant molecule).

## Claims

1. A cosmetic composition, **characterized in that** it comprises siliconed sapucainha ester and cosmetically acceptable vehicles and/or excipients, wherein the siliconed sapucainha ester is the heptamethyltrisiloxane-3- ethylcyclohexanol-(1-cyclopentenyl)-hexadecanoate ester.

2. A composition according to claim 1, **characterized by** comprising from 0.1% to 5.0% by weight of siliconed sapucainha ester.

3. A composition according to claim 1 or 2, **characterized by** comprising from 0.1% to 3.0% by weight of siliconed sapucainha ester.

4. A cosmetic product **characterized by** comprising the composition defined in any claims 1 to 3.

5. A product according to claim 4, **characterized by** being lipstick, waxy base, blusher, tinted base, facial powder or makeup product for the eye area.

## Patentansprüche

1. Kosmetische Zusammensetzung, **dadurch gekennzeichnet, dass** sie siliconisierten Sapucainha-Ester und kosmetisch annehmbare Stoffträger und/oder Hilfsstoffe umfasst, wobei es sich bei dem siliconisierten Sapucainha-Ester um Heptamethyltrisiloxan-3-ethylcyclohexanol-(1-cyclopentenyl)-hexadecanoatester handelt.

2. Zusammensetzung nach Anspruch 1, **dadurch gekennzeichnet, dass** sie 0,1 bis 5,0 Gew.-% an siliconisiertem Sapucainha-Ester umfasst.

3. Zusammensetzung nach Anspruch 1 oder 2, **dadurch gekennzeichnet, dass** sie 0,1 bis 3,0 Gew.-% an siliconisiertem Sapucainha-Ester umfasst.

4. Kosmetikprodukt, **dadurch gekennzeichnet, dass** es die in einem der Ansprüche 1 bis 3 definierte Zusammensetzung umfasst.

5. Produkt nach Anspruch 4, **dadurch gekennzeichnet, dass** es ein Lippenstift, eine wachshaltige Grundlage, ein Rouge, eine getönte Grundlage, ein Gesichtspuder oder ein Make-up-Produkt für den Augenbereich ist.

## Revendications

1. Composition cosmétique, **caractérisée en ce qu'**elle comprend un ester de sapucainha siliconé et des véhicules et/ou excipients acceptables du point de vue cosmétique, dans laquelle l'ester de sapucainha est l'ester heptaméthyltrisiloxanne-3-éthylcyclohexanol-(1-cyclopentényl)-hexadécanoate.

2. Composition suivant la revendication 1, **caractérisée en ce qu'**elle comprend 0,1% à 5,0 % en poids d'ester de sapucainha siliconé.

3. Composition suivant la revendication 1 ou 2, **caractérisée en ce qu'**elle comprend 0,1 % à 3,0 % en poids d'ester de sapucainha siliconé.

4. Produit cosmétique **caractérisé en ce qu'**il comprend la composition définie dans l'une quelconque des revendications 1 à 3.

5. Produit suivant la revendication 4, **caractérisé en ce qu'**il consiste en un rouge à lèvres, une base cireuse, un fard à joues, une base tintée, une poudre faciale ou un produit de maquillage pour la zone oculaire.
